# EUROPEAN PATENT APPLICATION

(11) **EP 3 849 279 A1**
(43) Date of publication of application: **14.07.2021**
(21) Application number: 20315004.0
(22) Date of filing: 08.01.2020
(51) Int. Cl.: H05B 45/12, A61B 5/16, A61B 5/00, G06F 3/13

(54) **HEAD UP DISPLAY WITH EYE TRACKING CAPABILITIES**

(71) Applicant: Ellcie Healthy, 06270 Villeneuve Loubet (FR)
(72) Inventor: PEYRARD, Philippe, 75015 Paris (FR)
(74) Representative: Ipside

(57) **Abstract**

The invention pertains to system for measuring a gaze direction of a user comprising a headpiece (100) featuring a display screen (111) set in front of the user eye and comprising a plurality of at least 3 emitters (131, 132, 133, 134, 135, 136) attached to the edges of the screen in separated locations, each IR emitter having a light emission cone and emitting a beam directed toward the eye of the user so as to create a lighting spot on the cornea, further comprising at least one IR receiver (142, 145) set on an edge of the screen, wherein the IR emitters are activated one at a time in sequence.

## Description

### Technical Field

The invention belongs to the field of wearable devices featuring eye tracking capabilities. Such eye tracking capabilities may be implemented in a viewfinder or a Head Up Display of a wearable device, where they may be used to control several functions piloted by the gaze of the wearer. Measuring and analysing the direction of the gaze of the wearer or its variation over time, may also be used, as such or in combination with other parameters, to assess the physical or mental state of the wearer such as stress or alertness.

### Background Art

A head up display or HUD is a screen in the form of a transparent LCD display set in front of one eye or both eyes of a user on which information is displayed, enabling the user to view that information while looking through the screen, and to take advantage of so called augmented reality.

Such a HUD is, as a non-limiting example, used by an aircraft pilot, where information such as altitude, speed, pitch and roll as well as maps are displayed, enabling the pilot to view that information without moving the head.

Providing eye tracking capabilities to such a device, enables the user to trigger actions with his eye, e.g. by looking at a specific area of the display, or by a movement of the eye, while keeping his hands on the commands.

An action may also be triggered by an eyeblink or successive eyeblinks of the user.

Examples of actions are numerous and to quote some non-limiting examples the user may, with his eye, simply switch on and switch off the display, change the displayed information by scrolling through pages or a drop-down menu, select a point to be followed/tracked, change the display conditions like the color or the luminosity of the screen.

Measuring the gaze direction may be performed either by a camera or by an infrared detector, in fact a plurality of infrared detectors. In both cases the eye is lit by an infrared source, and the detection is performed by the difference in reflectivity of the sclera, the iris and the pupil. Therefore, when the direction of the gaze changes the intensity of the reflected light is changed.

In order to actually detect the direction of the gaze, an image processing is performed in the case of a detection by a camera, or, when an infrared sensor is used, the lightening incident beam is focused in one or multiple, crosshairs like narrow bands, in combination with a plurality of detectors, thus allowing an accurate assessment of the gaze direction.

Typically, 4 band like lighting spots arranged as a cross, each of a rectangular shape of about 1 mm x 3 mm, and projected on the cornea, are used to this end.

However, those devices and methods of the prior art are implementing a large number of sensors and detectors and require quite a large computing power, leading to a high electrical power consumption when implemented in a wearable device.

Furthermore, because those devices are using narrow, crosshairs like lighting strips on the cornea, their accuracy may be influenced by vibrations, eyeblinks, facial expressions of the user or eyelashes intercepting or modifying the IR beams projected on the user eye.

In addition, when such a device is to be manufactured in large batches using e.g. plastics, the manufacturing tolerances do not allow to get a precise and adapted location of the light emitting spots as well as the sensors for a given user, right of the box. Therefore, such devices of the prior art require mechanical adjusting means, in order to calibrate them for each user.

Such mechanical calibration means are increasing the cost and the overall footprint of the device, are likely to loosen and require a complex calibration method involving a trained technician.

### Disclosure of Invention

It is an object of the invention to solve the disadvantages of the prior art and to provide a system capable of reliably assessing the gaze direction of a user, and optionally eyeblinks, with a limited set of sensors while being cost effective, reliable and robust vis-a-vis manufacturing tolerances when manufactured in large batches.

To this end the invention pertains to a system for measuring a gaze direction of a user comprising a headpiece featuring a display screen set in front of the user eye and comprising a plurality of at least 3 emitters attached to the edge of the screen in separated locations, each IR emitter having a light emission cone and emitting a beam directed toward the eye of the user so as to create a lighting spot on the cornea, further comprising at least one IR receiver set on an edge of the screen, wherein the IR emitters are activated one at a time in sequence.

As the IR emitters are located in separated locations on the edge of the screen, they emit with a different incident angle with respect to the surface of the eye, and, even with the use of a single detector, these differences in the reflected light seen from the IR receiver, combined with the sequential activation of the IR emitters allow to reliably measure the direction of the gaze while using wide lighting spots on the cornea. The wideness of the lighting spots minimizes the influence of the user morphology, of the manufacturing tolerances of the device and of vibrations on the results.

The accuracy and the effectiveness of the system may be further improved considering the following embodiments that can be implemented individually or in any combination thereof.

According to an embodiment the light emission cone of a first set of emitters is different than the light emission cone of a second set of emitters. The result is that the light spots projected by each set of emitters on the cornea are of different diameters thus increasing the change of the received reflected light seen from the IR receiver when the direction of the gaze changes.

According to that embodiment the headset comprises emitters projecting a light spot on the cornea comprised between 1.5mm and 6mm in diameter and at least one emitter projecting a light spot of 10mm in diameter. Therefore, the area of a light spot is at least 7mm² and up to 314mm², to be compared with the devices of the prior art where crosshairs light spots are each covering an area of about 6mm².

Advantageously, the angular wideness of the light emission cone at mid-intensity of emitters located in a lower part of the screen is comprised between 9° and 40°, and the angular wideness of the light emission cone at mid-intensity of emitters set in an upper part of the screen is equal or more than 70°.

The lighting spot generated by the IR emitter set in the lower part of the screen is less likely to be disturbed by eyelashes or some facial expressions, that location is therefore better suited for the narrow beam emission.

According to another embodiment, the IR emitters locations and orientations on the screen are set so that the lighting spots are projected in different areas of the cornea of the user eye.

According to another embodiment the intensity of the light emitted by a first emitter is set to a different value than the intensity of the light emitted by a second emitter.

Advantageously, the IR receiver is located beside an IR emitter and separated from that IR emitter by an optical barrier. This implementation avoids so called "cross talk" between the receiver and the emitter and improves the accuracy of the intensity measurement of the reflected beams, while also enabling a more compact design.

In a specific embodiment the headpiece is integrated to an audio headset.

In another specific embodiment the headpiece is integrated into a helmet.

### Brief Description of Drawings

The application is disclosed hereafter according to its preferred embodiments that are in no way limiting and with reference to figures 1 to 5, in which:
[fig. 1] Figure 1 shows in perspective views an exemplary embodiment of the device of the system of the invention;
[fig. 2] figure 2, shows according to a perspective view an example of the lighting spots projected on the eyes of the user according to an exemplary embodiment;
[fig. 3] figure 3, is an example of reflected patterns variation when the gaze is moved from left to right for a device using 4 IR emitters;
[fig 4] illustrates the setup used to calibrate the system of the invention;
[fig. 5] and figure shows an exemplary arrangement of the sensors with their connecting flexible PCB and an exemplary embodiment of a platelet adapted for holding sensors.

### Best Mode for Carrying Out the Invention

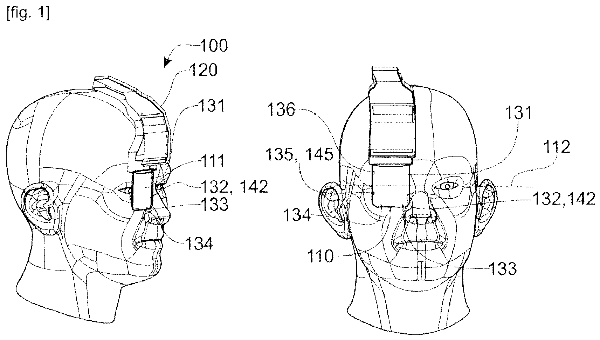

Figure 1, according to an exemplary embodiment the sensors of the system of the invention are borne by a headset (100) that may be integrated into a helmet such as a pilot helmet or to an audio headset like the one used by an helicopter pilot (not shown).

The headset features a screen (111) in the form of a transparent LCD on which information may be displayed.

A plurality of IR emitters (131, 132, 133, 134, 135, 136) are clipped on the edges of the screen. Those emitters are emitting IR beams towards the eye of the user. At least 3 IR emitters are set.

The emitters are distributed around the perimeter of the screen.

When only 3 IR emitters are used, they are distributed around the perimeter of the screen in a triangular shape arrangement.

Considering an imaginary (112) line passing through the center of the user's pupils and approximately at mid height of the screen, thus defining a lower part and an upper part of the screen, at least one IR emitter (131, 136) is set in the upper part of the screen.

At least one IR receiver (142, 145) is set on an edge of the screen beside an IR emitter (132, 135) and measures the intensity of the IR light projected by the emitters and reflected by the cornea of the user.

Setting the IR receiver beside an IR emitter reduces the footprint of the sensors in the user's field of view.

An IR emitter consists in a IR emitting LED featuring or not a focusing lens depending on its location on the edge of the screen.

The focusing lens reduces the wideness of the light emission cone of an emitter.

A bare emitter, without lens, exhibits a light emission cone at mid-intensity of about 70°, projecting a light spot of about 10mm in diameter on the cornea of the user.

Adding a lens reduces the angular wideness of the light emission cone to a value comprised between 9° and 40°, depending on the lens, the diameter of the light spot projected on the cornea being comprised between 1.5mm and 6mm respectively.

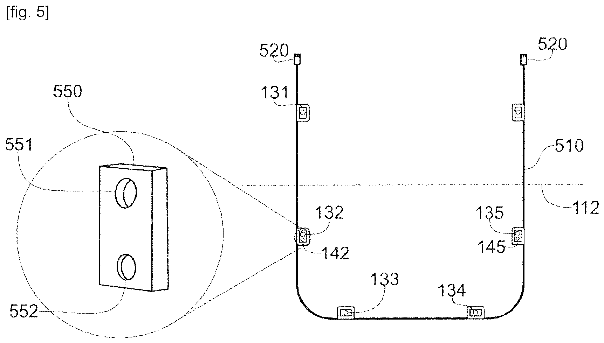

Figure 5, a thin flexible printed circuit board (PCB) (510) protected by a soft sheath is glued on the edges of the screen for supplying electrical power to the IR emitters and receivers and for collecting the signals of the IR receivers.

To this end the PCB (510) comprises connectors (520) to be connected in the electronic module of the headset.

According to an embodiment, the IR emitters are housed in platelets made of an opaque plastic material.

A specific platelet (550) suited to receive an IR emitter and an IR receiver features two hollow housings (551, 552) in which the IR receiver and the IR emitter are snapped.

The space between the two housings (551, 552) provides an optical barrier between the receiver and the emitter, thus avoiding "cross talk" where the direct light emitted by the emitter influences the measure of the reflected light by the receiver.

Depending on the application and the precision required for the eye tracking function, the device of the invention may feature up to 6 emitters and up to 4 receivers. It is however an objective of the invention to reduce the number of sensors used for a given precision.

To this end the following guidelines lead to the best result:

The device may feature at least 3 IR emitters and one IR receiver.

The IR emitters shall be distributed along the perimeter of the screen.

At last one IR emitter shall be located in the lower part of the screen, and at least one IR emitter shall be located in the upper part of the screen.

At least one IR emitter shall feature a focusing lens in order to project a narrower light spot on the cornea.

When only one of such emitters comprising a focusing lens is used, it shall be located in the lower part of the screen.

When the detection of eyeblinks is sought, the device shall feature at least one bare emitter projecting a wide spot, and when only one of such emitters is used, it shall be located in the upper part of the screen.

The receivers may be located in the upper part or the lower part of the screen, but when located in the lower part of the screen they should be placed as close as possible of the center line (112) as shown in figure 5.

Back to figure 1, the headset comprises an electronic module (120) including electronics to control the IR emitters, like their emission power or their switch on and switch off, to acquire the signal from the IR receiver and to make the appropriate signal analysis and calculations so as to assess the gaze direction.

To this end the electronic module comprises a microprocessor and memory means, a battery and power management means.

The electronic module may also feature communication means, such as a Bluetooth® module, enabling the device to exchange and receive data from a computer. Alternatively, or in addition, the electronic module may also feature a wire connection such as a micro-USB® connector for exchanging data with a computer.

As a result, using a device connected to the electronic module either through a wired or a wireless connection and using a suitable application, the conditions of light emission of each IR emitter may be changed, e.g. for a calibration purpose, as well as the parameters used to assess the gaze direction and stored in the memory means. Therefore, the device may be adapted to the user.

It is therefore an advantage of the system of the invention to be calibrated via a software, not requiring a complex mechanical setup.

According to an exemplary embodiment, 2 IR emitters (133, 134) are set in the lower part of the screen and are projecting a light spot on the user cornea.

In a preferred embodiment those 2 IR emitters consist in IR emitting LED and are equipped with a lens focusing the beam and reducing the light emission cone at mid-intensity in a range comprised between 9° and 40°, resulting in light spots with a diameter comprised between 1.5mm and 6mm depending on the focusing lens.

IR emitters (131, 136) are set on the upper part of the screen, and at least one of those IR emitters (131) does not feature a focusing lens and lights the eye with a beam having an emission cone at mid-intensity of about 70°, resulting in a lighting spot of 10mm.

Such a wide spot may also be used to detect eyeblinks by the difference of reflectivity between the cornea and the eyelid.

Detecting eyeblinks may be useful to trigger some specific actions by a voluntary eyeblink of the user, but also to discard inconsistent measurements of the gaze direction, e.g. because of an involuntary eyeblink disturbing the measure.

Eyeblinks detection and analysis, more specifically of involuntary eyeblinks, may also be used to measure the state of alertness of the user, and to detect a drop in its alertness.

Such a state of alertness measurement using eyeblinks pattens is disclosed in the document US10,152,869 B2.

According to an exemplary embodiment, IR emitting LEDs (132, 135) are set in the lower half part of the screen and are preferably equipped with a focusing lens in order to produce a reduced diameter light spot, comprised between 1.5mm and 6mm.

According to this embodiment, the IR receivers (142, 145) are set beside those emitters close to the mid-line (112).

The IR receivers (142, 145) measure the intensity of the IR light reflected by the whole eye of the user when lit by the IR emitters, either by the cornea area: sclera, iris or pupil or part thereof, when the eye is open, or by the eyelid when the eye is closed or partially closed.

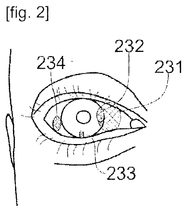

Figure 2 shows an exemplary combination of lighting spots on the user eye while the user is looking straightforward. In order to simplify the drawing only the spots of 4 IR emitters are displayed and the resulting information is described considering only one IR receiver;

According to this exemplary embodiment, the upper IR emitter (131 figure 1) projects a fairly wide spot (231) on the cornea.

The mid IR emitter (132 figure 1), projects a narrower spot (232) in the vicinity of the upper IR emitter spot.

These two light spots are lighting the left side of the eye.

A first lower IR emitter (134 figure 1) projects a narrow spot (234) on the right side of the eye.

A second lower IR emitter (133 figure 1) projects a narrower spot (233) in the centre of the lower part of the eye.

The person skilled in the art understands that this exemplary layout is not limitative and may be different depending on the application.

Starting from the situation of figure 2, if the user looks to the left, the eye iris will progressively penetrate in the wide spot (231) changing the reflected light since the reflection of the iris is different from the reflection of the sclera, while the spot (234) on the right side will still be reflected by the sclera. If the user looks further to the left, the pupil will cross the edge of the narrower spot (232) on the left side, also resulting in a change of the reflected light, while the spot (234) on the right side is still reflected by the sclera, and the spot (233) in the centre of the lower side is still mainly reflected by the iris.

Therefore, when the light reflected by the whole eye is measured, any position of the eye, thus any direction of the gaze, will result in a different reflected intensity.

In order to further improve the accuracy of the gaze direction measurement, the emitters are switched on an off in sequence.

As a for instance, the 4 IR emitters are switched on and switched off one by one according to the following sequence:
- Lighting the upper IR emitter
- Lighting the right lower emitter
- Lighting the middle emitter
- Lighting the left lower emitter

Each emitter is activated during a fraction of a second, ranging between 10⁻⁵ and 10⁻³ second, and the IR receiver measures the reflected intensity corresponding to the reflection of each emitter taken individually, thus giving the reflection intensity pattern consisting in a set of 4 reflection intensities for each gaze direction.

Each set of acquisitions is preferably performed at a frequency ranging from 50Hz to 200Hz, preferably 100Hz.

Such an acquisition frequency is much higher than any movement of the eye or any eyeblink frequency.

As an exemplary embodiment, patterns corresponding to specific gaze directions are stored in the memory means of the headset, the pattern measured by the IR receiver or receivers may thus be compared to those recorded patterns to assess the gaze direction.

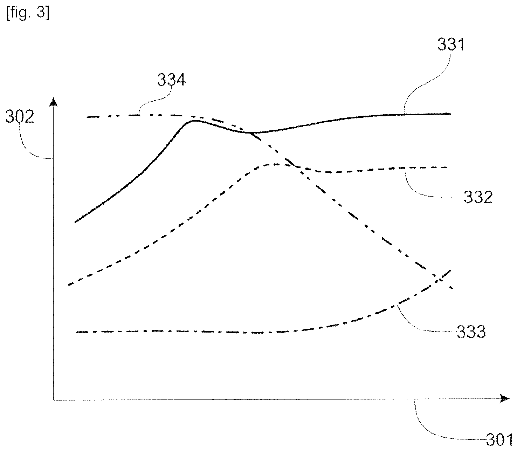

Figure 3, in an improved embodiment, in addition to specific reflection intensity patterns, intensity variation patterns are stored in the memory means for a plurality of eye movements.

As a for instance, figure 3 shows a variation of the reflected light intensity (302) according to the position (301) of the centre of the pupil of the eye when the gaze is moved from left to right, when the eye is lit by the upper LED (331), by the middle LED (332), the left lower LED (334) and the right lower LED (333).

The reflectivity of the eyelid is much higher than the reflectivity of any part of the sclera, the iris and the pupil. The duration of an involuntary eyeblink is comprised between 0.1 and 0.3 seconds. Therefore, when an involuntary eyeblink occurs during an acquisition sequence, it is easily detected, more specifically with the reflected light associated with the upper emitter projecting a wide spot, and processed accordingly, e.g. by discarding any corresponding gaze direction measurement.

In order for the gaze direction detection to work reliably it is better for the system to be calibrated individually, meaning for each pair of user/device.

Such a calibration makes it possible to correct variations in the manufacturing of the headset and in the IR emitting LED as well as IR receiver and allows to take into account the user morphology as well as its way of wearing the headset.

*The calibration method of the device is performed using a specific software and does not require any mechanical operation on the device.

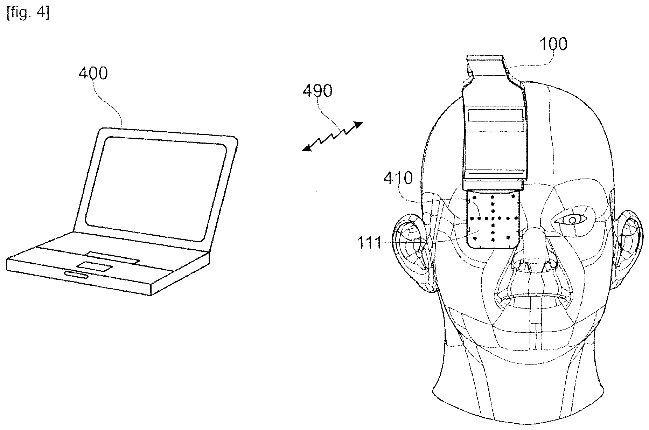

Figure 4, to this end, the headset (100) is connected to a computer (400) via a wired or a wireless (490) link.

The calibration is performed in two steps.

A first step aims at setting the light emission power of the IR emitters. The user is simply looking straightforward without blinking while wearing the headset. The LED are lit in sequence and the reflected intensity measured. Data are exchanged between the headset (100) and the computer (400).

An application installed in the computer compares the results to specific values and tolerances stored in a memory. If the measurements are laying in the correct range the calibration process moves to the next step.

If the measurements are outside the acceptable range, then the measurement are further analysed in order to detect a malfunctioning device, i.e. one of the LEDs or an emitter is not working properly or to adjust the light emitting power of the LEDs. The emission power of each LED may be set to a different value.

Once the first calibration step is performed a fine tuning of the system may be performed to improve the accuracy of the system.

To this end, according to an exemplary embodiment, a network of dots (410) is displayed on the screen (111) in known positions.

The user looks toward each dot (410) in a defined sequence, while the IR emitters are lit in sequence and the reflected light acquired by the IR receivers as in normal operation.

The procedure may be repeated several times.

Data are exchanged with the computer (400) and computed in order to define the reflected light patterns and patterns variation for the specific user.

That information is then uploaded in the memory means of the headset.

The description hereinabove and the exemplary embodiments show that the aim of the invention is reached and that the system of the invention enables to assess the gaze direction of a user while using a reduced number of low cost emitters and sensors.

The configuration of the lighting spots of the cornea, the acquisition sequence, triggering the IR emitters one at a time, as well as the simple calibration procedure make the system robust vis-a-vis manufacturing tolerances and the user morphology, while being cost effective.

## Claims

1. A system for measuring a gaze direction of a user comprising a headpiece (100) featuring a display screen (111) set in front of the user eye and comprising a plurality of at least 3 emitters (131, 132, 133, 134, 135, 136) attached to the edges of the screen in separated locations, each IR emitter having a light emission cone and emitting a beam directed toward the eye of the user so as to create a lighting spot (331, 332, 333, 334) on the cornea, further comprising at least one IR receiver (142, 145) set on an edge of the screen, wherein the IR emitters are activated one at a time in sequence.

2. The system of claim 1, wherein the light emission cone of a first set of emitters is different than the light emission cone of a second set of emitters.

3. The system of claim 2, wherein the headset (100) comprises emitters (132, 133, 134, 135) projecting a light spot on the cornea comprised between 1.5mm and 6mm in diameter and at least one emitter (131) projecting a light spot of 10mm in diameter.

4. The system of claim 2, comprising at least one IR emitter (132, 133, 134, 135) located in a lower part of the screen (111) and at least one IR emitter (131, 136) located in an upper part of the screen, wherein , the angular wideness of the light emission cone at mid-intensity of emitters (132, 133, 134, 135) located in the lower part of the screen (111) is comprised between 9° and 40°, and the angular wideness of the light emission cone at mid-intensity of one emitter (131) set in an upper part of the screen is equal or more than 70°.

5. The system of claim 1, wherein the IR emitters locations and orientations around the screen are set so that the lighting spots (331, 332, 333, 334) are projected in different areas of the cornea of the user eye.

6. The system of claim 1, wherein the intensity of the light emitted by a first emitter is set to a different value than the intensity of the light emitted by a second emitter.

7. The system of claim 1, wherein the IR receiver (142, 145) is located beside an IR emitter (132, 135) and separated from that IR emitter by an optical barrier.

8. The system of claim 1, wherein the headpiece (100) is integrated in a helmet.

9. The system of claim 1, wherein the headpiece (100) is integrated to an audio headset
